# EUROPEAN PATENT APPLICATION

(11) **EP 3 988 389 A1**
(43) Date of publication of application: **27.04.2022**
(21) Application number: 21204065.3
(22) Date of filing: 21.10.2021
(51) Int. Cl.: B60N 2/815, B60N 2/818, B60N 2/844, B60N 2/847

(54) **HEADREST ASSEMBLY WITH SLIDABLE RATCHET MECHANISM**

(30) Priority: 22.10.2020 US 202063104210 P
(71) Applicant: Windsor Machine and Stamping (2009) Ltd., Windsor, Ontario N9C 2L8 (CA)
(72) Inventor: Angelo, Collins, Windsor, ON (CA); Little, Mark, Windsor, ON (CA)
(74) Representative: Manitz Finsterwald Patent- und Rechtsanwaltspartnerschaft mbB

(57) **Abstract**

A headrest assembly for use in a vehicle includes a housing having a front frame (22) and a rear frame (20), the front frame (22) slidably connected to the rear frame (20). The headrest assembly also includes an armature operatively coupling the rear portion to a seatback of an automobile seat. The headrest assembly further includes an adjustment mechanism permitting movement of the front frame (22) with respect to the rear frame (20) from a vertically non-adjusted position, wherein the front frame (22) is generally vertically aligned with the rear frame (20), to a vertically adjusted position, wherein the front frame (22) is moved vertically from the vertically non-adjusted position. Upon actuation of the assembly, disengaging vertical adjustment, also an inclination adjustment is disengaged at the same time.

## Description

### CROSS-REFERENCE TO RELATED APPLICATION

This application claims priority to U.S. Provisional Patent Application Serial No. 63/104,210, filed October 22, 2020, the disclosure of which is incorporated by reference herein in its entirety.

### FIELD OF THE INVENTION

The present invention relates to vehicle headrests and, more particularly, to a headrest assembly that is adjustable via a slidable ratchet mechanism.

### BACKGROUND

This section provides background information related to the present disclosure which is not necessarily prior art.

Technological improvements of automobiles have generally been focused on categorical enhancements to efficiency, safety, and comfort. Oftentimes, an improvement in one category detracts from one of the other categories. For example, there has been a consistent struggle throughout the historical development of automobiles to find a balance between strength and weight. While incorporating stronger components may ultimately lead to a safer driving experience, stronger materials are typically heavier than other materials and thus detract from operational efficiency, e.g., fuel economy. Similarly, a focus primarily on efficiency can lead to components that are vulnerable to accidents and normal wear and tear.

One example component of an automobile that has historically been developed with an emphasis on strength and comfort is a headrest. Most automobiles include headrests atop an occupant's seat and in a position adjacent the occupant's head. Because headrests are specifically designed to interface with an occupant's head, they must be comfortable both tactilely and positionally. In addition to comfort, headrests must be able to withstand amounts of impact to prevent whiplash to the occupant during a rear end collision, to a certain extent, block foreign objects in the event of a crash or sudden braking situation. Developments in comfort functionality and, more particularly, positional adjustment for both safety and comfort have resulted in headrests having adjustment mechanisms that are relatively complicated to operate.

Accordingly, there is a continued desire to develop headrests that provide positional comfort, that are strong, and that are simple to use.

### SUMMARY OF THE DISCLOSURE

This section provides a general summary of the disclosure and is not to be interpreted as a complete and comprehensive listing of all of the objects, aspects, features and advantages associated with the present disclosure.

In accordance with one aspect of the disclosure, a headrest assembly for use in a vehicle includes a housing having a front portion and a rear portion, the front portion slidably connected to the rear portion. The headrest assembly also includes an armature operatively coupling the rear portion to a seatback of an automobile seat. The headrest assembly further includes an adjustment mechanism permitting movement of the front portion with respect to the rear portion from a vertically non-adjusted position, wherein the front portion is generally vertically aligned with the rear portion, to a vertically adjusted position, wherein the front portion is moved vertically from the vertically non-adjusted position.

Further areas of applicability will become apparent from the description provided herein. The description and specific examples in this summary are intended for purposes of illustration only and are not intended to limit the scope of the present disclosure.

### BRIEF DESCRIPTION OF THE DRAWINGS

The drawings described herein are for illustrative purposes only of selected embodiments and are not intended to limit the scope of the present disclosure. The inventive concepts associated with the present disclosure will be more readily understood by reference to the following description in combination with the accompanying drawings wherein:
Figure 1 is a front perspective view of a headrest assembly for an automobile in a vertically non-adjusted position;
Figure 2 is front perspective view of the headrest assembly in a vertically adjusted position wherein a front portion is vertically moved relative to a rear portion;
Figure 3 is side view of the headrest assembly in the vertically non-adjusted position;
Figure 4 is side view of the headrest assembly in the vertically adjusted position;
Figure 5 is a front perspective view of the headrest assembly illustrating a front frame and a rear frame in the vertically adjusted position;
Figure 6 is a rear perspective view of the front frame and the rear frame in the vertically adjusted position;
Figure 7 is a rear perspective view of the front frame and the rear frame in the vertically non-adjusted position;
Figure 8 is a perspective view of the rear frame wherein the front frame has been removed and exposes an adjustment mechanism;
Figures 9A through 9C are a series of cross-sectional views illustrating the movement of the front frame from the vertically non-adjusted position to the vertically adjusted position;
Figures 10A through 10C are a series of cross-sectional view illustrating certain components of the adjustment mechanism being moved from a locked position to a released position;
Figures 11A through 11D are a series of cross-sectional views illustrating the movement of the front frame and rear frame from a non-pivoted position to a pivoted position:
Figure 12A is a font perspective view of the rear frame;
Figure 12B is a rear perspective view of the front frame;
Figure 12C is an enlarged front perspective view of the rear frame;
Figure 13A is a cross-sectional view of a track system slidably connecting the front frame to the rear frame, wherein the front portion is in the vertically non-adjusted position;
Figure 13B is an enlarged view of the track system from Figure 13A;
Figure 14A is a cross-sectional view of the track system, wherein the front portion is in the vertically adjusted position;
Figure 14B is an enlarged view of the track system from Figure 14A;
Figure 15A is a cross-sectional view of a stopping mechanism of the track system, wherein the front portion is in the vertically non-adjusted position;
Figure 15B is an enlarged view of the stopping mechanism from Figure 14A;
Figure 16A is a cross-sectional view of the stopping mechanism, wherein the front portion is in the vertically adjusted position and continued vertical travel in the same direction is prevented by a stop;
Figure 16B is an enlarged view of the stopping mechanism from Figure 16B;
Figure 17A is a front perspective view of the headrest assembly in the vertically non-adjusted position and the non-pivoted position;
Figure 17B is a front perspective view of the headrest assembly in the vertically non-adjusted position and the pivoted position; and
Figure 17C is a front perspective view of the headrest assembly in the vertically adjusted position and the pivoted position.

### DETAILED DESCRIPTION

Example embodiments will now be described more fully with reference to the accompanying drawings. In general, the subject embodiments are directed to a headrest assembly for an automobile that is adjustable via a slidable ratchet mechanism. However, the example embodiments are only provided so that this disclosure will be thorough, and will fully convey the scope to those who are skilled in the art. Numerous specific details are set forth such as examples of specific components, devices, and methods, to provide a thorough understanding of embodiments of the present disclosure. It will be apparent to those skilled in the art that some specific details need not be employed, that example embodiments may be embodied in many different forms and that neither should be construed to limit the scope of the disclosure. In some example embodiments, well-known processes, well-known device structures, and well-known technologies are not described in detail.

Referring to the Figures, wherein like numerals indicate corresponding parts throughout the views, the headrest assembly is intended for providing an enhanced travel experience to occupants of an automobile by allowing the headrest assembly to be vertically adjusted and pivoted in the fore-aft direction.

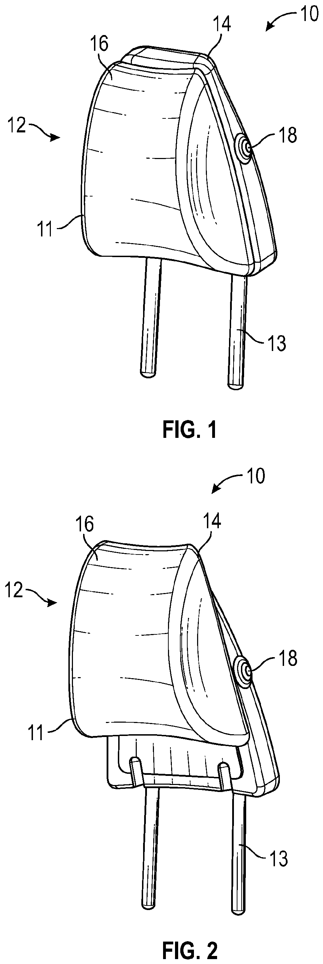

Referring to Figure 1, a headrest assembly is depicted. The headrest assembly is generally referred to with numeral 10. The headrest assembly 10 includes a housing 12 that at least partially encloses several components associated with stabilization and/or adjustment of the headrest assembly 10. A cushion and a cover 11 may be provided over the housing 12 for aesthetic purposes and occupant comfort, i.e., tactile comfort. A base portion 13 (which may also be referred to as "armature") is mountable to an automobile seat and, more specifically, to a top surface of a seatback of the automobile seat (not shown). The housing 12 includes a rear portion 14 and a front portion 16. The rear portion 14 is connected to the armature 13 and the front portion 16 is connected to the rear portion 14 such that vertical movement of armature 13 moves both the rear portion 14 and front portion 16. The headrest assembly 10 in Figure 1 is shown in a vertically non-adjusted position, wherein the front portion 16 is located in a default position with respect to the rear portion 14 such that the front portion 16 is vertically generally aligned therewith.

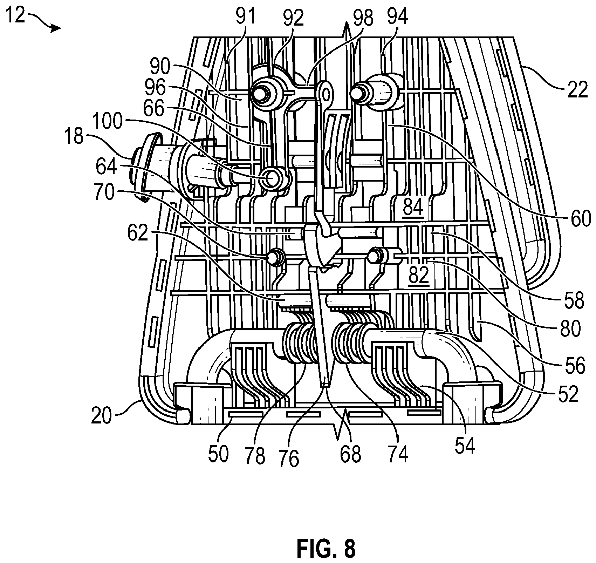

As best illustrated in Figures 2, the headrest assembly 10 includes a vertically adjusted position wherein the front portion 16 is moved vertically from the vertically non-adjusted position with respect to the rear portion 14. The vertical movement of the front portion 16 with respect to the rear portion 14 may be limited to positions above the vertically non-adjusted position, below the vertically non-adjusted position, or not limited. The extent and direction of movement of the front portion 16 may vary depending upon the particular application of use. As will be described in greater detail below, a slidable ratchet mechanism 15 (Figure 8) or, more generally, an adjustment mechanism 15 permits movement of the front portion 16. A button 18 located outside of the housing 12 and cover 11 is in operable communication with the adjustment mechanism 15 and effectuates switching between a locked condition, wherein downward movement of the front portion 16 is prevented and an unlocked or released condition, wherein movement of the front portion 16 is permitted in both directions. The button 18 may be connected to the rear portion 14.

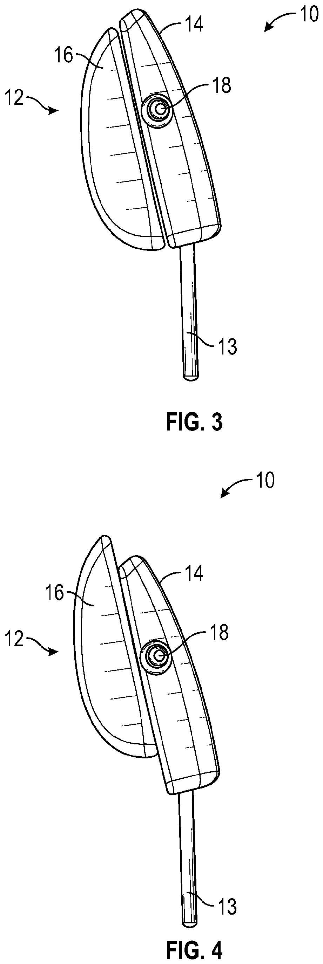

Figure 3 presents a side view of the headrest assembly in a vertically non-adjusted position, wherein the front portion is substantially vertically aligned with the rear portion 14. Figure 4 presents a side view of the headrest assembly 10 in a vertically adjusted position, wherein the front portion 16 has been moved upwardly with respect to the rear portion 14. Movement of the front portion 16 from the vertically non-adjusted position to the vertically adjusted position may be sliding movement.

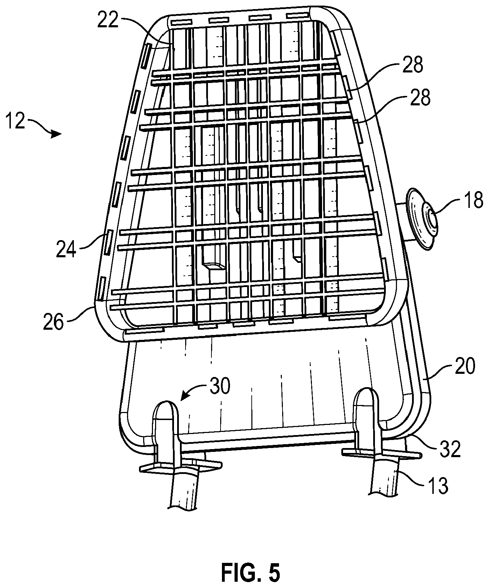

The housing 12 includes an interior frame that is illustrated from a front perspective view in Figure 5. More particularly, the rear portion 14 includes a rear frame 20 and the front portion 16 includes a front frame 22. The front frame 22 includes a forward facing front surface 24 that includes an outer rim 26 extending therearound. A webbing 28 extends across the forward facing front surface 24 between opposite sides of the outer rim 26. The rear frame 20 includes a forward facing rear surface 30 that includes a pair of ports 32 for entry of the armature 13.

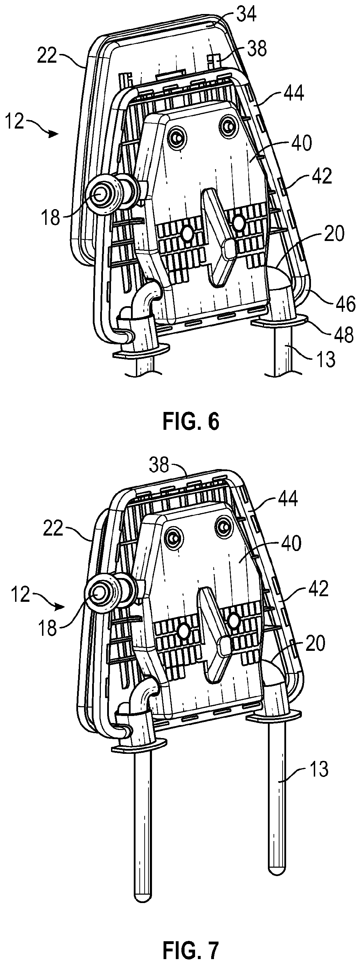

Figure 6 is a rear perspective view of the interior frame. The front frame 22 further includes a back facing front surface 34 and the rear frame 20 further includes a back facing rear surface 38. A rear shell 40 is connected to the back facing rear surface 38 for covering and locating certain components of the adjustment mechanism 15 (Figure 8). The rear shell 40 defines a rear bulged portion 42 for accommodating movement of certain components of the adjustment mechanism 15 during movement of the headrest assembly 10. The rear shell 40 includes a series of apertures 44 for receiving fasteners during assembly. The ports 32 include a sleeve section 46 that surrounds portions of the armature 13 and a base section 48 that tapers outwardly and contacts the seatback.

Figure 8 is another rear perspective view of the interior frame, wherein the rear shell 40 has been removed from the rear portion 14 to expose the adjustment mechanism 15. The back facing rear surface 38 includes a pair of hooks 50 that are spaced apart in the cross-car direction for placing a cross-car portion 52 of the armature 13 therein. Each hook 50 defines a lower bearing surface 54 that is in relative rotational contact with the cross-car portion 52 of the armature 13 as the headrest assembly 10 is pivoted between a non-pivoted position and a pivoted position. The back facing rear surface 38 further defines a includes a number of projections to hold various components of the adjustment mechanism 15. More particularly, the back facing rear surface 38 includes a first pin holding bracket 56, a second pin holding bracket 58 located vertically above the first pin holding bracket 56, and a third pin holding bracket 60 located vertically above the second pin holding bracket 58.

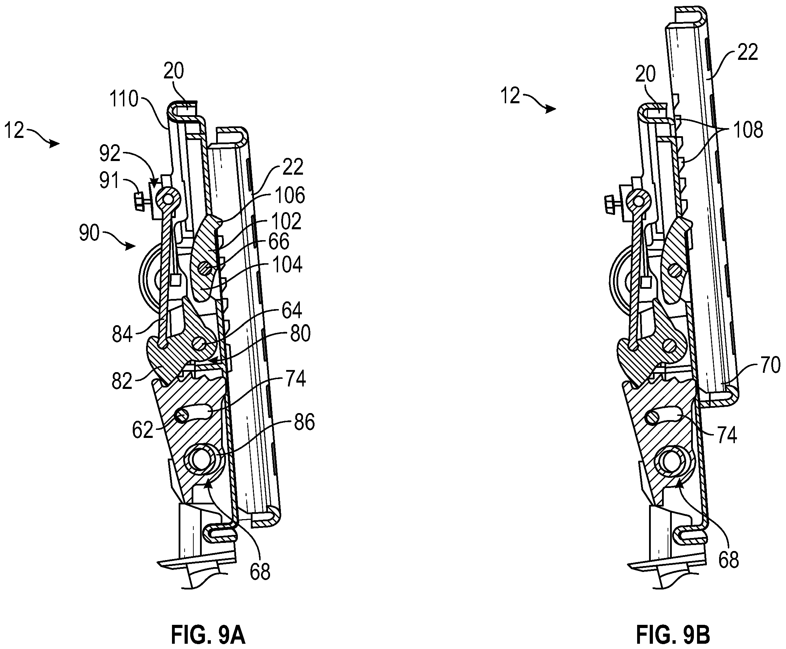

The adjustment mechanism 15 includes a first pivot pin 62 located in the first pin holding bracket 56, a second pivot pin 64 located in the second pin holding bracket 58, and a third pivot pin 66 located within the third pin holding bracket 60. A fixed ratchet member 68 is statically connected to the armature 13. The fixed ratchet member 68 includes a ratchet surface defining a plurality of fixed ratchet teeth 70. The fixed ratchet member 68 further includes an elongated opening 74 and a tail 76. The first pivot pin 62 extends through the elongated opening 74 of the fixed ratchet member 68. At least one spring 78 biases the first pivot pin 62 in a forward direction with respect to the elongated opening 74. A pair or torsion springs 78 are located on either side of the fixed ratchet member 68. Each torsion spring 78 includes an extended portion that presses on the tail 76 of the fixed ratchet member 68 and an extended portion on the opposite end that presses against the first pivot pin 62. The adjustment mechanism 15 further includes a moveable ratchet member 80 pivotally connected to the second pivot pin 64 that includes a ratchet surface defining at least one moveable ratchet tooth 82 engaged with the fixed ratchet teeth 70 on the fixed ratchet member 68. The moveable ratchet member 80 further includes a pivot arm 84 and a connection aperture 86 (Figures 9A and 9B) located between the pivot arm 84 and the moveable ratchet tooth 82. The moveable ratchet member 80 further includes a pivot pin opening 88 for receiving the second pivot pin 64 to allow it to pivot therearound.

With continued reference to Figure 8, a lever mechanism 90 connects to the connection aperture 86 and effectuates pivotal movement of the moveable ratchet member 80 with respect to the second pivot pin 64. The pivotal movement of the moveable ratchet member 80 brings the moveable ratchet tooth 82 into and out of engagement with the fixed ratchet teeth 70. The lever mechanism 90 includes a lever portion 92 and a pull member 94, wherein the pull member 94 connects to the connection aperture 86 of the moveable ratchet member 80 and the lever portion 92 is operably connected to the button 18. The lever portion 90 includes an angled body having a push section 96 and a pull section 98. The lever portion 90 is pivotally connected to a lever pin 91 that is located between the push section 96 and the pull section 98. The button 18 includes a plunger 100 that moves axially in response to the button 18 being manually pushed and resultantly pushes the lever portion 92 to effectuate upward pulling movement of the pull section 98, the pull member 94, and the moveable ratchet member 80 to disengage the moveable ratchet tooth 82 from the fixed ratchet teeth 70. When the moveable ratchet tooth 82 is engaged with the fixed ratchet teeth 70, ratcheting movement is allowed in one direction such that the moveable ratchet tooth 82 is directionally locked against subsequent fixed ratchet teeth 70. As such, the housing 12 can be adjusted/tilted in the fore-aft direction and locked into a number of tilt angles.

As best illustrated in Figure 9A and Figure 9B, a pawl 102 is connected to the third pivot pin 66 and includes a cam portion 104 and a pawl tooth portion 106. The front frame 22 further includes a rack of teeth 108 located on the back facing front surface 34 that is operably engaged with the pawl tooth portion 106. The cam portion 104 is in operable connection to the pivot arm 84 of the moveable ratchet member 80. As such, as the moveable ratchet member 80 pivots in response to actuation via the button 18, the pivot arm 84 contacts and pushes the cam portion 104 of pawl 102 and disengages the pawl tooth portion 106 from the rack of teeth 108. The rack of teeth 108 may be vertically arranged with respect to the seat. Engagement between the pawl tooth portion 106 and the rack of teeth 108 permits ratcheting movement in one direction such that the pawl tooth portion 106 is directionally locked against subsequent teeth in the rack of teeth 108. As such, the front portion 16 can be adjusted vertically in the fore-aft direction and locked into a number of vertical locations. A lever spring 110, such as a torsion spring, biases the lever mechanism 90 such that the moveable ratchet tooth 82 is biased into engagement with the fixed ratchet member teeth 70 and the pivot arm 84 of the moveable ratchet member 80 is not disengaging the pawl 102 from the rack of teeth 108. One or more springs (not shown) may further bias the pawl 102 into engagement with the rack of teeth 108. In Figure 9A, the front portion 16 is located in the vertically non-adjusted position and in Figure 9B the front portion 16 is located in the vertically adjusted position.

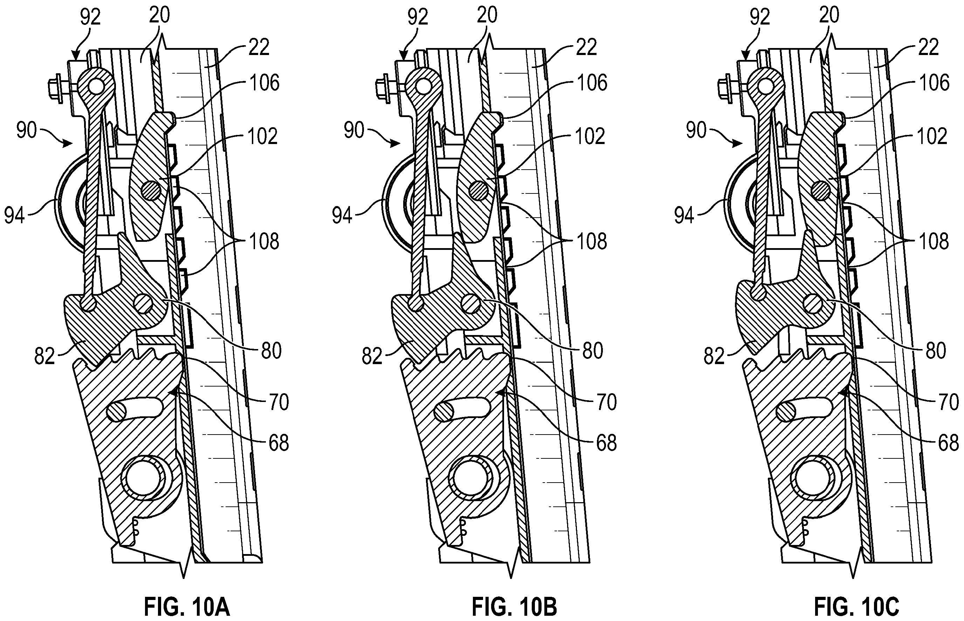

Figures 10A through 10C illustrate disengagement of the moveable ratchet tooth 82 from the fixed ratchet member teeth 70. In Figure 10A, the moveable ratchet tooth 82 and the fixed ratchet member teeth 70 are interconnected. In Figure 10B, the button 18 has been partially pressed such that the moveable ratchet member 80 has partially pivoted out of engagement with the fixed ratchet member 68. In Figure 10C, the button 18 has been fully pressed such that the moveable ratchet member 80 has pivoted out of engagement with the fixed ratchet member 68, thus allowing the housing 12 to move back in the fore direction. Figure 10C also illustrates that the pivoting of the moveable ratchet member 80 also causes the pivot arm 84 to disengage the pawl tooth portion 106 from the rack of teeth 108 and allow the front portion 16 to be moved downwardly. It should be appreciated that the direction of ratcheting lock could be any direction for both the vertical ratcheting and the pivoting ratcheting configurations.

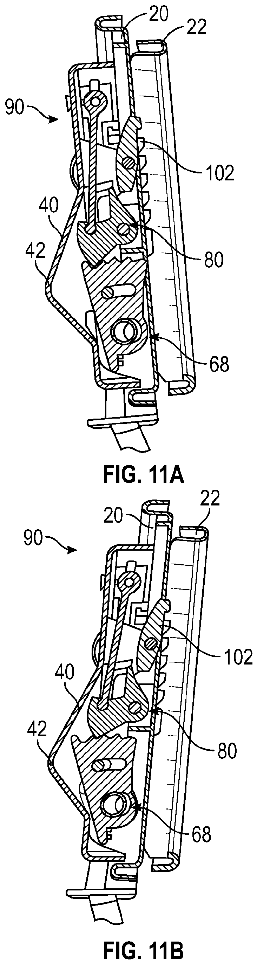

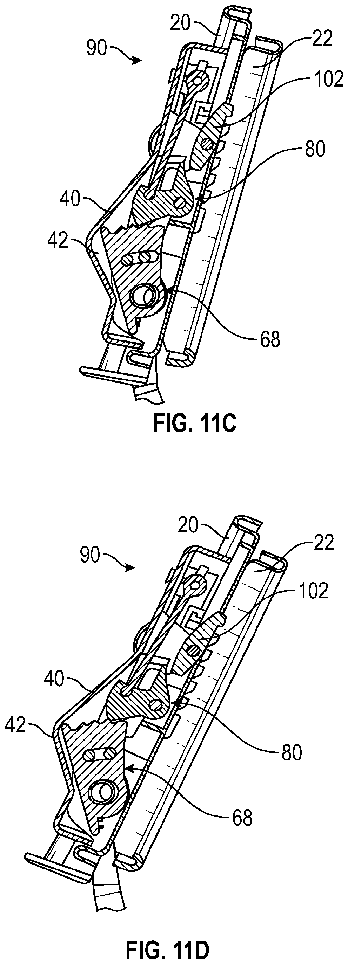

Figures 11A through 11D illustrate engagement of the moveable ratchet tooth 82 and the fixed ratchet member teeth 70 at various pivot angles with respect to the seat. In Figure 11A, the headrest assembly 10 is shown in the non-pivoted position wherein the housing 12 is generally aligned with the seat. Figure 11B illustrates the headrest assembly 10 in the pivoted position wherein the housing 12 is tilted in the fore direction. Figure 11C illustrates the headrest assembly 10 in the pivoted position wherein the housing 12 is tilted even further in the fore direction. Figure 11D illustrates the headrest assembly 10 in the pivoted position wherein the housing 12 is tilted even further yet in the fore direction. The rear bulged portion 42 accommodates movement of moveable ratchet member 80 in Figures 11A through 11D.

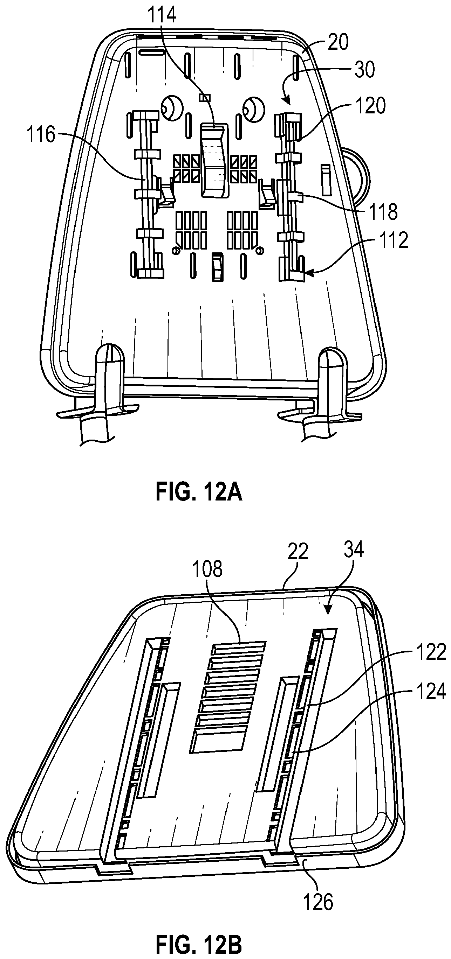

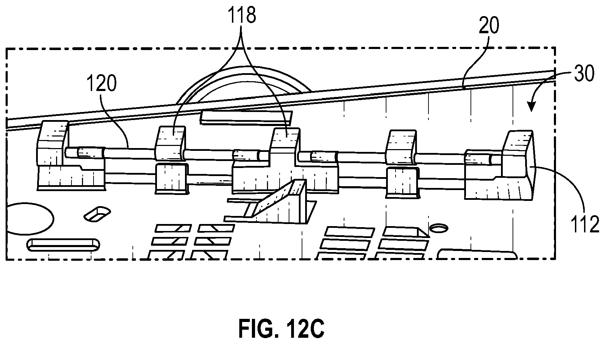

Figures 12A through 12C illustrate a track system that connects the front frame 22 to the rear frame 20. With reference initially to Figure 12A, the forward facing rear surface 30 includes a pair of vertical brackets 112 and a pawl opening 114 located between each of the vertical brackets 112. The pawl opening 114 accommodates movement of the pawl 102 into and out of engagement with the rack of teeth 108. The forward facing rear surface 30 also includes a pair of stops 116 for limiting the vertically adjusted position. The vertical brackets 112 each include pin holding clips 118 (Figure 12C) and a vertical pin 120 that is press fit between the holding clips. With reference now to Figure 12B, the back facing front surface 34 of the front frame 22 defines a pair of bracket grooves 122 for accommodating the brackets 112 and a pair of stop grooves 124 for accommodating the stops 116. The bracket grooves 122 each include a groove overhang 126 that is interlocked with one of the brackets 112 and in sliding contact with the vertical pin 120 between the vertically non-adjusted position and the vertically adjusted position. During movement between the vertically non-adjusted position and the vertically adjusted position, the stops 116 are permitted to move within the boundary of the stop grooves 124.

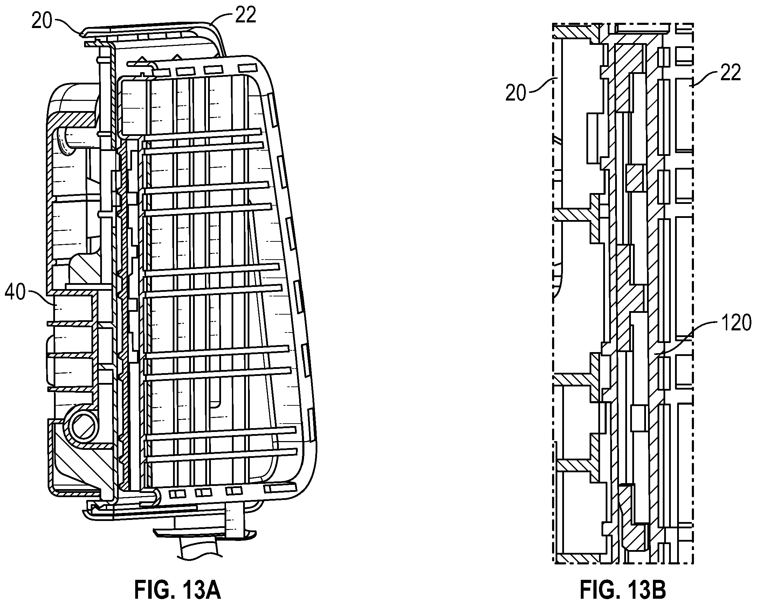

Figure 13A and Figure 13B are cross-sectional perspective views illustrating the connection between the bracket grooves 122 and the brackets 112 in the vertically non-adjusted position. Figure 13B is an enlarged view of Figure 13A.

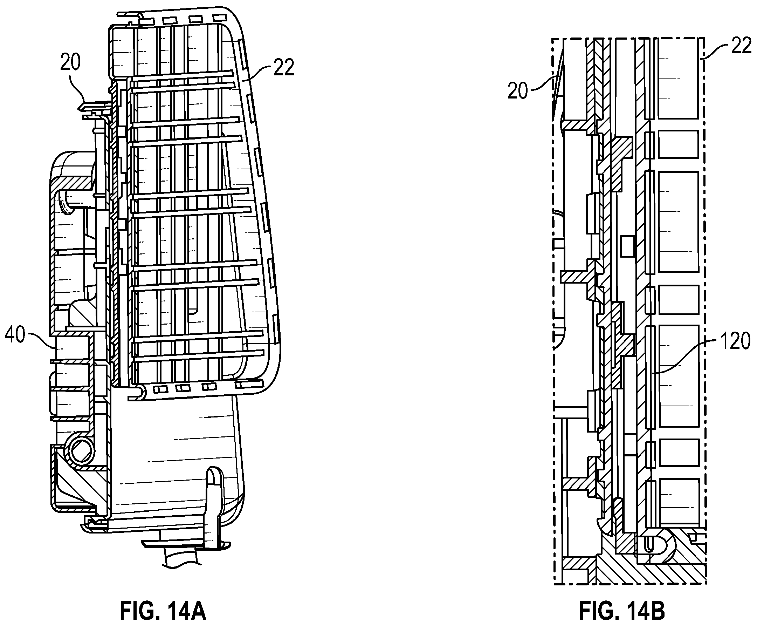

Figure 14A and Figure 14B are cross-sectional perspective views illustrating the connection between the bracket grooves 122 and the brackets 112 in the vertically adjusted position. Figure 14B is an enlarged view of Figure 14A.

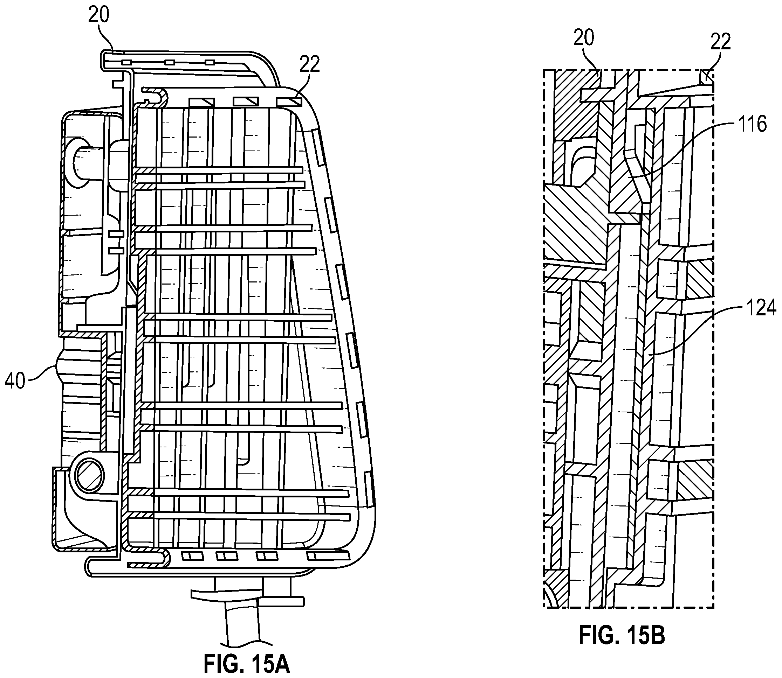

Figure 15A and Figure 15B are cross-sectional perspective views illustrating the connection between the stop grooves 124 and the stops 116 in the vertically non-adjusted position. Figure 15B is an enlarged view of Figure 15A.

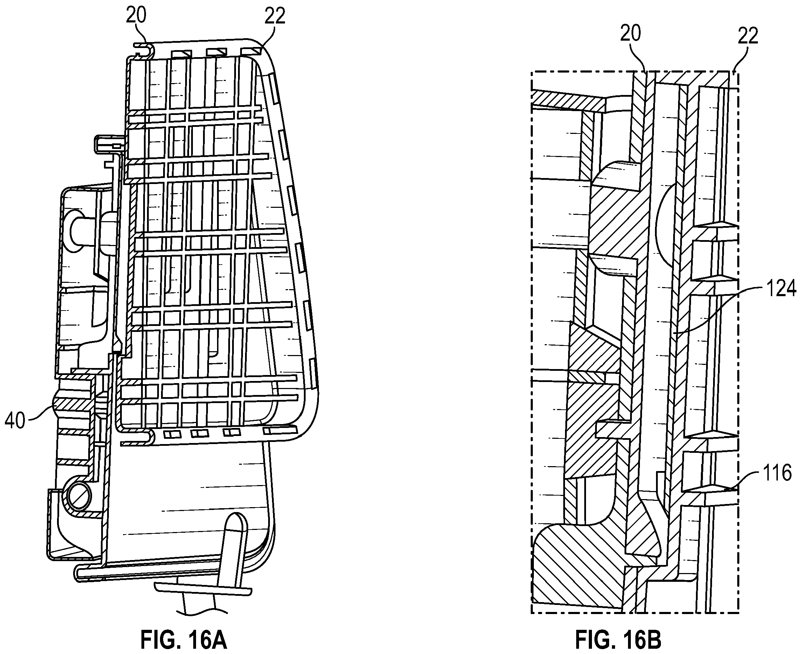

Figure 16A and Figure 16B are cross-sectional perspective views illustrating the connection between the stop grooves 124 and the stops 116 in the vertically adjusted position, wherein continued vertical movement of the front frame 22 in the same direction is prevented via contact with the stop 116 and the boundary of the stop groove 124. Figure 16B is an enlarged view of Figure 16A.

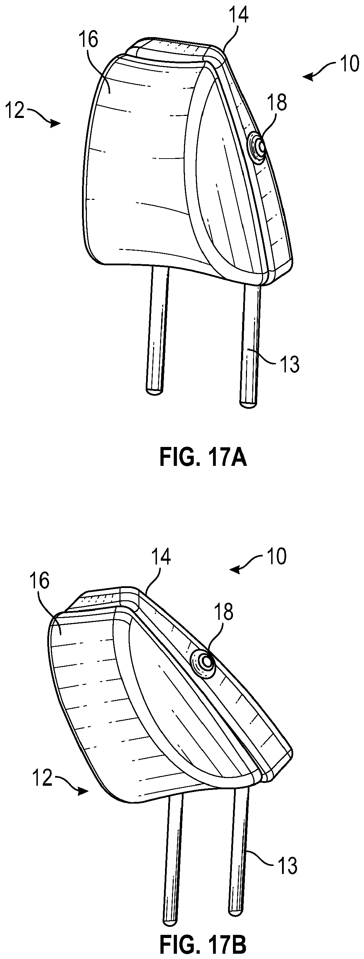

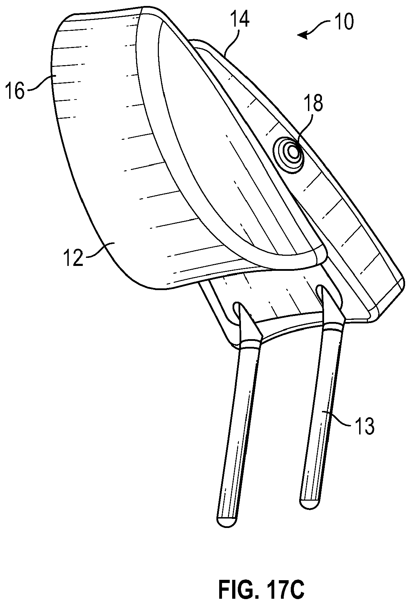

Figures 17A through 17B illustrate various positions of the headrest assembly 10 with a combination of both vertical movement of the front portion 16 and pivotal movement of the housing 12. Figure 17A illustrates the headrest assembly 10 in a vertically non-adjusted position and non-pivoted position. Figure 17B illustrates the headrest assembly 10 in a vertically non-adjusted position and pivoted position. Figure 17C illustrates the headrest assembly 10 in a vertically adjusted position and pivoted position.

While the invention has been described in detail in connection with only a limited number of embodiments, it should be readily understood that the invention is not limited to such disclosed embodiments. Rather, the invention can be modified to incorporate any number of variations, alterations, substitutions or equivalent arrangements not heretofore described, but which are commensurate with the spirit and scope of the invention. Additionally, while various embodiments of the invention have been described, it is to be understood that aspects of the invention may include only some of the described embodiments. Accordingly, the invention is not to be seen as limited by the foregoing description, but is only limited by the scope of the appended claims.

## Claims

1. A headrest assembly for use in a vehicle comprising:
a housing having a front portion and a rear portion, the front portion slidably connected to the rear portion;
an armature operatively coupling the rear portion to a seatback of an automobile seat; and
an adjustment mechanism permitting movement of the front portion with respect to the rear portion from a vertically non-adjusted position, wherein the front portion is generally vertically aligned with the rear portion, to a vertically adjusted position, wherein the front portion is moved vertically from the vertically non-adjusted position.

2. The headrest assembly of claim 1, wherein the adjustment mechanism comprises a pawl pivotally connected to the rear portion and a rack of teeth connected to the front portion, wherein the rack of teeth is positioned to engage with the pawl to place the headrest assembly in a locked condition of the front portion at a selected vertical position.

3. The headrest assembly of claim 2, wherein the adjustment mechanism further comprises:
a lever arrangement;
a moveable ratchet member coupled to the lever arrangement for pivoting movement of the moveable ratchet member; and
a fixed ratchet member positioned to be engaged with the moveable ratchet member to define a locked condition of the headrest assembly at a selected pivoted position.

4. The headrest assembly of claim 3, wherein the moveable ratchet member is positioned to pivot the pawl toward the unlocked condition of the vertical position upon movement of the moveable ratchet member.

5. The headrest assembly of claim 3, wherein the lever arrangement is operatively coupled to a button that is accessible to a user to move the adjustment mechanism to the unlocked condition.

6. The headrest assembly of claim 1, wherein the rack of teeth are formed on a rear face of the front portion.

7. The headrest assembly of claim 1, further comprising at least one stop feature disposed on a forward face of the rear portion to limit vertical movement of the front portion.

8. The headrest assembly of claim 1, wherein the pawl is disposed within a recess of a rear frame of the rear portion.

9. The headrest assembly of claim 1, further comprising a pair of rails disposed on a front face of the rear portion to guide vertical movement of the front portion.
